# EUROPEAN PATENT SPECIFICATION

(11) **EP 4 161 634 B1**
(45) Date of publication and mention of the grant of the patent: **21.02.2024**
(21) Application number: 21732596.8
(22) Date of filing: 28.05.2021
(51) Int. Cl.: A61N 1/39

(54) **AUTOMATED EXTERNAL DEFIBRILLATOR AND METHOD FOR NOTIFICATION ABOUT ABNORMALITY OF AUTOMATED EXTERNAL DEFIBRILLATOR**
AUTOMATISIERTER EXTERNER DEFIBRILLATOR UND VERFAHREN ZUR BENACHRICHTIGUNG ÜBER EINE ANOMALIE EINES AUTOMATISIERTEN EXTERNEN DEFIBRILLATORS
DÉFIBRILLATEUR EXTERNE AUTOMATISÉ ET PROCÉDÉ DE NOTIFICATION CONCERNANT UNE ANOMALIE D'UN DÉFIBRILLATEUR EXTERNE AUTOMATISÉ

(30) Priority: 05.06.2020 JP 2020098727
(43) Date of publication of application: 12.04.2023
(73) Proprietor: Nihon Kohden Corporation, Shinjuku-ku, Tokyo 161-8560 (JP)
(72) Inventor: KUMAMOTO, Kota, Tokorozawa-shi, Saitama 359-0037 (JP); IWAI, Fumihito, Tokorozawa-shi, Saitama 359-0037 (JP); AKIYAMA, Naoto, Tokorozawa-shi, Saitama 359-0037 (JP); KUNO, Ryosuke, Tokorozawa-shi, Saitama 359-0037 (JP)
(74) Representative: Grünecker Patent- und Rechtsanwälte PartG mbB
(86) International application number: PCT/JP2021/020431
(87) International publication number: WO 2021/246327

(56) References cited:
- US-A1- 2008 132 973
- US-A1- 2011 213 433
- US-B2- 9 808 639

## Description

### Technical Field

The present disclosure relates to an automated external defibrillator and a method for notification about an abnormality of the automated external defibrillator.

### Background Art

Generally, automated external defibrillators (hereinafter also abbreviated to AEDs) are mounted with self-test functions. In such a self-test, for example, a remaining level of a battery, a connection status with a defibrillation pad, whether various circuits operate normally or not, etc. are checked so that it is determined whether the AED can be used normally or not.

When an abnormality has been found by the self-test, the AED displays presence of the abnormality or generates an alert sound for notification of the abnormality. In this manner, the AED notifies a user of the abnormality. For example, Patent Literature 1 discloses a technique in which a user is notified of an abnormality of an AED by both display using an LED (Light Emitting Diode) and an alert sound.

### Citation List

### Patent Literature

PTL 1: U.S. Unexamined Patent Application Publication 2011/0213433

### Summary of Invention The invention is defined in claims 1 and 6.

### Technical Problem

In order to make a user around the AED easily notice the abnormality of the AED, it is preferable that both a visible alert and an audible alert are performed, as disclosed in Patent Literature 1. On the other hand, when both the visible alert and the audible alert are simultaneously performed, there is a possibility that an instantaneous load on a battery may increase.

An object of the present disclosure is to provide an automated external defibrillator that can suppress a load on a battery while notifying a user of an abnormality by a visible and audible method when the abnormality has been found in the automated external defibrillator, and a method for notification about the abnormality of the automated external defibrillator.

### Solution to Problem

According to a first aspect of the present disclosure, there is provided an automated external defibrillator including:
a detector that detects an abnormality of the automated external defibrillator; and
a notifier that notifies a user of the detection of the abnormality within a predetermined period when the detector has detected the abnormality; wherein:
   the notifier has a display unit that includes a light source, and a sounder that generates an alert sound;
   the display unit executes a first mode and a second mode alternately and one or more times respectively, so that the display unit makes the light source emit light with a first intensity in the first mode, and the display unit either makes the light source emit light with a second intensity lower in power consumption than the first intensity, or makes the light source not emit light in the second mode;
   the sounder executes a third mode and a fourth mode alternately and one or more times respectively, so that the sounder generates the alert sound with a third intensity in the third mode, and the sounder either generates the alert sound with a fourth intensity lower in power consumption than the third intensity, or does not generate the alert sound in the fourth mode;
   at least a half part of each of periods in which the first mode is executed overlaps timewise with a corresponding one of periods in which the fourth mode is executed; and
   at least a half part of each of periods in which the third mode is executed overlaps timewise with a corresponding one of periods in which the second mode is executed.

According to a second aspect of the present disclosure, there is provided a method for notification about an abnormality of an automated external defibrillator, the method making the automated external defibrillator execute
a detection step of detecting an abnormality of the automated external defibrillator; and
a notification step of notifying a user of the detection of the abnormality within a predetermined period when the abnormality has been detected in the detection step;
wherein:
   the notification step has a displaying step of making a display by a light source, and a sounding step of generating an alert sound;
   the displaying step is a step of executing a first mode and a second mode alternately and one or more times respectively, so that the light source is made to emit light with a first intensity in the first mode, and the light source is either made to emit light with a second intensity lower in power consumption than the first intensity, or made not to emit light in the second mode;
   the sounding step is a step of executing a third mode and a fourth mode alternately and one or more times respectively, so that the alert sound is generated with a third intensity in the third mode, and the alert sound is either generated with a fourth intensity lower in power consumption than the third intensity, or not generated in the fourth mode;
   at least a half part of each of periods in which the first mode is executed overlaps timewise with a corresponding one of periods in which the fourth mode is executed; and
   at least a half part of each of periods in which the third mode is executed overlaps timewise with a corresponding one of periods in which the second mode is executed.

### Advantageous Effects of Invention

According to the aforementioned configuration, it is possible to suppress a load on a battery while notifying a user of an abnormality of an automated external defibrillator by a visible and audible method when the abnormality has been found in the automated external defibrillator.

### Brief Description of Drawings

[Fig.1] FIG. 1 is a block diagram illustrating an example of a configuration of an automated external defibrillator according to an embodiment of the present disclosure.
[Fig.2A]FIG. 2A is a schematic diagram illustrating a first action example of the automated external defibrillator according to the embodiment of the present disclosure.
[Fig.2B]FIG. 2B is a schematic diagram illustrating details of a portion X illustrated in FIG. 2A.
[Fig.3A]FIG. 3A is a schematic diagram illustrating a second action example of the automated external defibrillator according to the embodiment of the present disclosure.
[Fig.3B]FIG. 3B is a schematic diagram illustrating a third action example of the automated external defibrillator according to the embodiment of the present disclosure.
[Fig.3C]FIG. 3C is a schematic diagram illustrating a fourth action example of the automated external defibrillator according to the embodiment of the present disclosure.
[Fig.3D]FIG. 3D is a schematic diagram illustrating a fifth action example of the automated external defibrillator according to the embodiment of the present disclosure.

### Description of Embodiments

An embodiment of the present disclosure will be described below by way of example with reference to the drawings. In the following description, identical or equivalent elements will be designated by the same reference signs or names correspondingly and respectively even in different drawings, and duplicate description thereof will be therefore omitted appropriately.

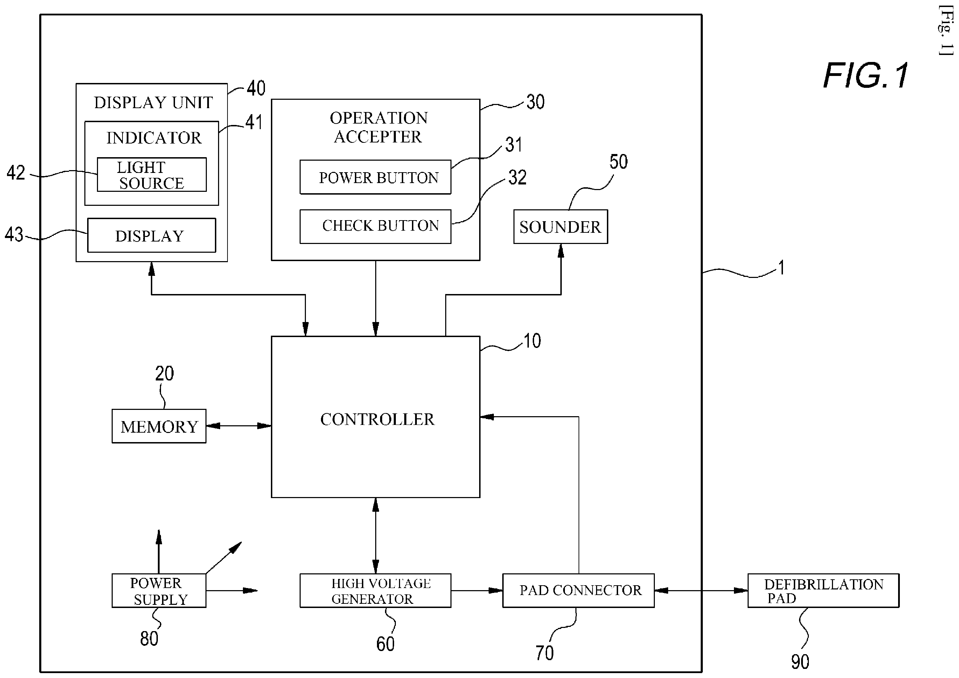

First, respective processors constituting an AED 1 will be described using FIG. 1. FIG. 1 is a block diagram illustrating an example of a configuration of the AED 1 according to the embodiment of the present disclosure. The AED 1 is provided with a controller 10, a memory 20, an operation accepter 30, a display unit 40, a sounder 50, a high voltage generator 60, a pad connector 70 and a power supply 80.

The controller 10 reads and executes a program etc. stored in the memory 20 to control various actions of the AED 1. The controller 10 has a processor such as a CPU (Central Processing Unit), a memory such as an ROM (Read Only Memory) or an RAM (Random Access Memory), a real time clock, an A/D converter, etc.

The controller 10 controls various actions for life rescue (hereinafter also referred to as "life rescue actions") such as energy charging/discharging control, sequence control, A/D conversion, and electrocardiogram analysis. In addition, the controller 10 executes a self-test to detect an abnormality of the AED 1. That is, the controller 10 also functions as a detector. The self-test may be executed when a setting time instant set in advance came, or when the operation accepter 30 has accepted a predetermined operation input (e.g. a check button 32 which will be described later has been pressed down).

In the self-test, for example, the controller 10 performs check of circuits for controlling life rescue actions (such as confirmation of a time constant of an electrocardiogram input circuit, confirmation of a circuit recognizing paddle contact, and confirmation of an energy value during charging into a capacitor/during internal discharging), check of the power supply 80 (such as a voltage value, a value of a remaining level of a battery, and a value of current consumption), check of a defibrillation pad 90 connected to the pad connector 70 (such as a resistance value of the pad and confirmation of expiry date for use), etc. to confirm whether they are normal or abnormal.

The memory 20 stores a program necessary for the AED 1 to act, audio data, an adjustment value, electrocardiogram data during the life rescue, a result of the self-test, etc. The memory 20 may include, for example, a secondary memory device such as a hard disk. A part of the memory 20 may be an external memory device that can be detachably attached to the AED 1.

The operation accepter 30 accepts an operation input from a user. The operation accepter 30 includes a power button 31 and the check button 32. The power button 31 is a button for starting a life rescue action. The check button 32 is a button for starting the self-test. In addition, although not illustrated, the operation accepter 30 may be provided with a shock button for executing an electric shock, a button for setting a setting time instant of the self-test, etc.

The display unit 40 includes an indicator 41 and a display 43. The display 43 is, for example, a liquid crystal display. The display 43 displays an instruction to the user as a figure or characters or displays an electrocardiogram signal. The display 43 may be provided with a touch panel or may function also as the operation accepter 30.

The indicator 41 has one or more light sources 42. Each of the light sources 42 is, for example, an LED light source. The light source 42 may include a fluorescent substance. The indicator 41 uses a lighting form (such as a color or blinking) of each of the light sources 42 to display a state of the AED 1. When no abnormality has been detected in the AED 1 by the self-test, it is determined that the AED 1 is normal. For example, a color such as a green color or a blue color may be displayed on the indicator 41.

When an abnormality has been detected in the AED 1, the display unit 40 functions as a part of a notifier that notifies the user of the detection of the abnormality within a predetermined period. Description will be made in detail in the following paragraphs in a case where the display unit 40 functions as the notifier.

The sounder 50 issues various instructions to the user by voice with reference to the audio data stored in the memory 20. In addition, when the abnormality has been detected in the AED 1, the sounder 50 functions as a part of the notifier that notifies the user of the detection of the abnormality within the predetermined period. Description will be made in detail in the following paragraphs in a case where the sounder 50 functions as the notifier.

The high voltage generator 60 carries out charging and discharging of energy used for defibrillation in accordance with a control signal from the controller 10. The pad connector 70 is connected to the defibrillation pad 90. The energy discharged by the high voltage generator 60 is transmitted to a person in need for rescue through the pad connector 70 and the defibrillation pad 90. In addition, the defibrillation pad 90 fetches an electrocardiogram signal of the person in need for rescue. The electrocardiogram signal is, for example, filtered and amplified before being transmitted to the controller 10.

The power supply 80 includes the battery. The power supply 80 converts electric power supplied from the battery into a required voltage, and supplies the electric power to the aforementioned processors. The remaining level of the battery can be confirmed by the self-test.

An action example of the AED 1, particularly an abnormality notification method by the AED 1 will be described below. The AED 1 executes a detection step of detecting an abnormality of the AED 1 by a detector (the controller 10), and a notification step of notifying a user of the detection of the abnormality within a predetermined period when the abnormality has been detected in the detection step. The aforementioned "predetermined period" is not limited particularly. For example, the "predetermined period" is about two to three seconds. It is preferable that the notification step is repeatedly executed in a predetermined cycle (e.g. every thirty seconds) unless a suspension condition in which the abnormality of the AED 1 has been cancelled or the user has performed an operation for suspending the notification is satisfied.

The notification step includes a displaying step of making a display by the light sources 42 on the indicator 41, and a sounding step of generating an alert sound by the sounder 50. The displaying step is a step in which a first mode and a second mode are executed alternately based on an instruction signal issued from the controller 10. In the first mode, the light sources 42 are made to emit light with a first intensity. In the second mode, the light sources 42 are either made to emit light with a second intensity lower in power consumption than the first intensity, or made not to emit light.

Here, the first intensity is not limited particularly if it is an intensity with which the light emitted by the light sources 42 can be visibly recognized by the user. The second intensity is not limited particularly if it is an intensity lower in power consumption than the first intensity. However, it is preferable that the power consumption for the second intensity is as low as possible. The light emitted by the light sources 42 with the second intensity may be visibly unrecognizable by the user. It is preferable that the second mode is a mode in which the light sources 42 are made not to emit light.

In the displaying step, each of the first mode and the second mode is executed one or more times, preferably executed a plurality of times. The number of times the first mode is executed and the number of times the second mode is executed may be the same as each other or may be different from each other. At least a half part of each of periods in which the first mode is executed overlaps timewise with a corresponding one of periods in which a fourth mode which will be described later is executed by the sounder 50. Preferably at least a nine tenth part of the period in which the first mode is executed, more preferably the whole of the period in which the first mode is executed overlaps timewise with the period in which the fourth mode which will be described later is executed.

The sounding step is a step in which a third mode and the fourth mode are executed alternately based on an instruction signal issued from the controller 10. In the third mode, an alert sound is generated with a third intensity. In the fourth mode, the alert sound is either generated with a fourth intensity lower in power consumption than the third intensity, or not generated.

Here, the third intensity is not limited particularly if it is an intensity with which the alert sound can be recognized by the user. The fourth intensity is not limited particularly if it is an intensity lower in power consumption than the third intensity. However, it is preferable that the power consumption for the fourth intensity is as low as possible. The alert sound generated with the fourth intensity may be unable to be recognized by the user. It is preferable that the fourth mode is a mode in which the alert sound is not generated.

In the sounding step, each of the third mode and the fourth mode is executed one or more times, preferably executed a plurality of times. The number of times the third mode is executed and the number of times the fourth mode is executed may be the same as each other or may be different from each other. At least a half part of each of periods in which the third mode is executed overlaps timewise with a corresponding one of periods in which the second mode is executed by the display unit 40. Preferably at least a nine tenth part of the period in which the third mode is executed, more preferably the whole of the period in which the third mode is executed overlaps timewise with the period in which the second mode is executed.

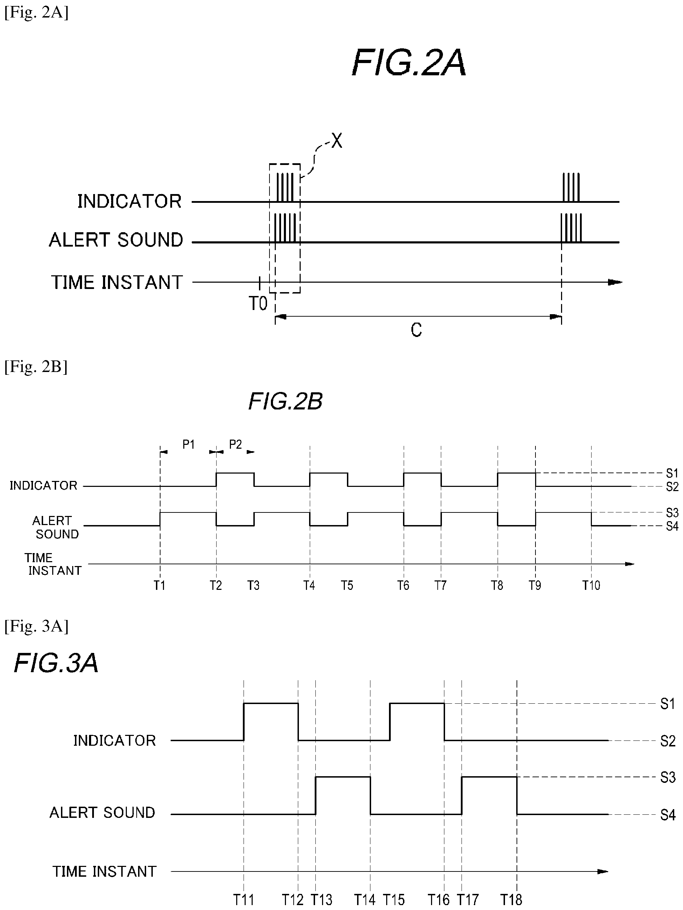

Action examples of the AED 1 will be specifically described below using FIGS. 2A and 2B and FIGS. 3A to 3D. FIG. 2A is a schematic diagram illustrating a first action example of the AED 1. In FIG. 2A, a time instant T0 is a time instant at which an abnormality is detected in the AED 1. Each bar line extending vertically from an axis described as "indicator" means that the light sources 42 emit light inside the indicator 41. In a similar manner or the same manner, each bar line extending vertically from an axis described as "alert sound" means that the sounder 50 generates an alert sound.

When the abnormality has been detected at the time instant T0, a notification step is executed as illustrated in a portion X. Specifically, the light sources 42 emit lights four times and the sounder 50 generates the alert sound five times. This notification step is repeatedly executed in the cycle C unless the aforementioned suspension condition is satisfied.

FIG. 2B is a schematic diagram illustrating details of the portion X illustrated in FIG. 2A. In FIG. 2B, time instants T1 to T10 are a period in which the notification step is executed. An intensity S1 denotes a first intensity. An intensity S2 denotes that the intensity is zero, i.e. a state in which the light sources 42 are turned off. An intensity S3 denotes a third intensity. An intensity S4 denotes that the intensity is zero, i.e. a state in which the alert sound is not generated. Incidentally, intensities S1 to S4 in other drawings are also similar or the same.

In a period P1 illustrated between the time instants T1 and T2, the display unit 40 executes the second mode, and the sounder 50 executes the third mode. That is, in the period P1, the sounder 40 generates the alert sound while the light sources 42 are turned off. Each of periods illustrated between the time instants T3 and T4, between the time instants T5 and T6, between the time instants T7 and T8, and between the time instants T9 and T10 is also similar to or the same as the period P1.

In a period P2 illustrated between the time instants T2 and T3, the display unit 40 executes the first mode, and the sounder 50 executes the fourth mode. That is, in the period P2, the sounder 40 does not generate the alert sound while the light sources 42 are turned on. Each of periods illustrated between the time instants T4 and T5, between the time instants T6 and T7, and between the time instants T8 and T9 is also similar to or the same as the period P2. Accordingly, in the example of FIG. 2B, each period in which the first mode is executed, and each period in which the third mode is executed do not overlap timewise with each other.

A length of each of the period P1 and the period P2 is not limited particularly. For example, the length of the period P1 and P2 is preferably not more than 1,000 milliseconds, and may be not more than 300 milliseconds. In addition, the lengths of the period P1 and the period P2 may be the same as each other or may be different from each other. In other words, the length of the period in which the first mode is executed and the length of the period in which the second mode is executed may be the same as each other or may be different from each other. In a similar manner or the same manner, the length of the period in which the third mode is executed and the length of the period in which the fourth mode is executed may be the same as each other or may be different from each other.

In addition, the periods in each of which the first mode is executed may be different in length from one another. For example, the period between the time instants T2 and T3 may be 100 milliseconds, and the period between the time instants T4 and T5 may be 200 milliseconds. A similar rule or the same rule thing may also apply to the periods in which the second to fourth modes are executed.

FIG. 3A is a schematic diagram illustrating a second action example of the AED 1. In FIG. 3A, the display unit 40 executes the first mode in a period illustrated between time instants T11 and T12 and a period illustrated between time instants T15 and T16. In a similar manner or the same manner, the display unit 40 executes the second mode in a period illustrated between the time instants T12 and T15 and a period illustrated between the time instants T16 and T18.

In addition, the sounder 50 executes the third mode in a period illustrated between time instants T13 and T14 and a period illustrated between time instants T17 and T18. In a similar manner or the same manner, the sounder 50 executes the fourth mode in a period illustrated between the time instants T11 and T13, and a period illustrated between the time instants T14 and T17.

In the example of FIG. 3A, each of the periods in which the second mode is executed and each of the periods in which the fourth mode is executed overlap timewise with each other. In other words, the period in which the notification step is executed contains periods in each of which the light sources 42 are turned off and the alert sound is also not generated.

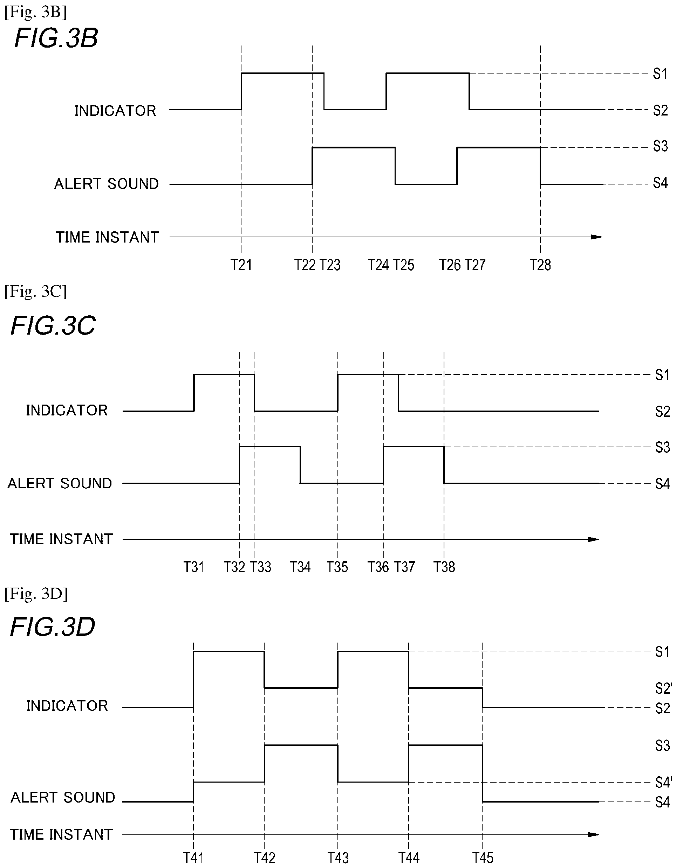

FIG. 3B is a schematic diagram illustrating a third action example of the AED 1. In FIG. 3B, the display unit 40 executes the first mode in a period illustrated between time instants T21 and T23 and a period illustrated between time instants T24 and T27. In a similar manner or the same manner, the display unit 40 executes the second mode in a period illustrated between the time instants T23 and T24 and a period illustrated between the time instants T27 and T28.

The sounder 50 executes the third mode in a period illustrated between time instants T22 and T25 and a period illustrated between time instants T26 and T28. In a similar manner or the same manner, the sounder 50 executes the fourth mode in a period illustrated between the time instants T21 and T22 and a period illustrated between the time instants T25 and T26.

In the example of FIG. 3B, each of the periods in which the first mode is executed and each of the periods in which the third mode is executed overlap timewise with each other. In other words, the period in which the notification step is executed contains periods (between the time instants T22 and T23, between the time instants T24 and T25, and between the time instants T26 and T27) in each of which the light sources 42 are turned on and the alert sound is also generated.

At least a half part of each of the periods in which the first mode is executed overlaps timewise with each of the periods in which the fourth mode is executed. Specifically, a length between the time instants T21 and T22 is at least a half part of a length between the time instants T21 and T23. In a similar manner or the same manner, a length between the time instants T25 and T26 is at least a half part of a length between the time instants T24 and T27.

In a similar manner or the same manner, at least a half part of each of the periods in which the third mode is executed overlaps timewise with each of the periods in which the second mode is executed. Specifically, a length between the time instants T23 and T24 is at least a half part of a length between the time instants T22 and T25. In a similar manner or the same manner, a length between the time instants T27 and T28 is at least a half part of a length between the time instants T26 and T28.

FIG. 3C is a schematic diagram illustrating a fourth action example of the AED 1. In FIG. 3C, the display unit 40 executes the first mode in a period illustrated between time instants T31 and T33 and a period illustrated between time instants T35 and T37. In a similar manner or the same manner, the display unit 40 executes the second mode in a period illustrated between the time instants T33 and T35 and a period illustrated between the time instants T37 and T38.

In addition, the sounder 50 executes the third mode in a period illustrated between time instants T32 and T34 and a period illustrated between time instants T36 and T38. In a similar manner or the same manner, the sounder 50 executes the fourth mode in a period illustrated between the time instants T31 and T32 and a period illustrated between the time instants T34 and T36.

In the example of FIG. 3C, the periods in which the second mode is executed and the periods in which the fourth mode is executed overlap timewise with each other (between the time instants T34 and T35) in a similar manner to or the same manner as the example of FIG. 3A.

In addition, in the example of FIG. 3C, the periods in which the first mode is executed and the periods in which the third mode is executed overlap timewise with each other (between the time instants T32 and T33 and between the time instants T36 and T37) in a similar manner to or the same manner as the example of FIG. 3B.

At least a half part of each of the periods in which the first mode is executed overlaps timewise with each of the periods in which the fourth mode is executed. Specifically, a length between the time instants T31 and T32 is at least a half part of a length between the time instants T31 and T33. In a similar manner or the same manner, a length between the time instants T35 and T36 is at least a half part of a length between the time instants T35 and T37.

In a similar manner or the same manner, at least a half part of each of the periods in which the third mode is executed overlaps timewise with each of the periods in which the second mode is executed. Specifically, a length between the time instants T33 and T34 is at least a half part of a length between the time instants T32 and T34. In a similar manner or the same manner, a length between the time instants T37 and T38 is at least a half part of a length between the time instants T36 and T38.

FIG. 3D is a schematic diagram illustrating a fifth action example of the AED 1. An intensity S2' illustrated in FIG. 3D is a second intensity. That is, the intensity S2' means a state in which each of the light sources 42 is turned on to emit light with an intensity lower than a state of an intensity S1. In addition, an intensity S4' is a fourth intensity. That is, the intensity S4' means a state in which the sounder 50 generates the alert sound with a sound volume lower than a state of an intensity S3.

In FIG. 3D, the display unit 40 executes the first mode in a period illustrated between time instants T41 and T42 and a period illustrated between time instants T43 and T44. In a similar manner or the same manner, the display unit 40 executes the second mode, i.e. the light sources 42 emit light in a darker state than that in the first mode, in a period illustrated between the time instants T42 and T43 and a period illustrated between the time instants T44 and T45. In addition, the light sources 42 are turned off in a period before the time instant T41 and a period after the time instant T45.

In addition, the sounder 50 executes the third mode in the period illustrated between the time instants T42 and T43 and the period illustrated between the time instants T44 and T45. In a similar manner or the same manner, the sounder 50 executes the fourth mode, i.e. the sounder 50 generates the alert sound with a sound volume lower than that in the third mode, in the period illustrated between the time instants T41 and T42 and the period illustrated between the time instants T43 and T44. In addition, the alert sound is not generated in the period before the time instant T41 and the period after the time instant T45.

Successively, effects obtained by the respective configurations included in the AED 1 and the abnormality notification method of the AED 1 according to the present embodiment will be described.

At least a half part of each of the periods in which the first mode large in power consumption is executed overlaps timewise with a corresponding one of the periods in which the fourth mode small in power consumption is executed, and at least a half part of each of the periods in which the third mode large in power consumption is executed overlaps timewise with a corresponding one of the periods in which the second mode small in power consumption is executed. With the configuration made thus, an instantaneous load on the battery can be suppressed while the user is notified of an abnormality of the AED 1 by a visible and audible method.

In addition, at least a nine tenth part of each of the periods in which the first mode is executed overlaps timewise with a corresponding one of the periods in which the fourth mode is executed, and at least a nine tenth part of each of the periods in which the third mode is executed overlaps timewise with a corresponding one of the periods in which the second mode is executed. With the configuration made thus, an instantaneous load on the battery can be suppressed more greatly.

In addition, each of the periods in which the first mode large in power consumption is executed and each of the periods in which the third mode large in power consumption is executed do not overlap timewise with each other. With the configuration made thus, an instantaneous load on the battery can be suppressed further greatly.

In addition, the second mode is set as a mode in which the light sources are made not to emit light, and the fourth mode is set as a mode in which the alert sound is not generated. The fourth mode is executed in each of the periods in which the first mode is executed, and the second mode is executed in each of the periods in which the third mode is executed. With the configuration made thus, an instantaneous load on the battery can be suppressed further greatly.

In addition, when each of the periods in which the first to fourth modes are executed is set to have a length not more than 1,000 milliseconds, a visible alert and an audible alert are repeated at short time intervals. Accordingly, the user can be more easily made aware of the presence of the abnormality.

The aforementioned embodiment is merely exemplary in order to make the presently disclosed subject matter easy to understand. The configuration according to the aforementioned embodiment can be changed/improved properly without departing from the scope of the presently disclosed subject matter. The present application is based on Japanese Patent Application No. 2020-098727 filed on June 5, 2020.

### Reference Signs List

1: automated external defibrillator (AED)
10: controller (detector)
20: memory
30: operation accepter
31: power button
32: check button
40: display unit (notifier)
41: indicator
42: light source
43: display
50: sounder (notifier)
60: high voltage generator
70: pad connector
80: power supply
90: defibrillation pad

## Claims

1. An automated external defibrillator (1) comprising:
a detector (10) adapted to detect an abnormality of the automated external defibrillator; and
a notifier adapted to notify a user of the detection of the abnormality within a predetermined period when the detector has detected the abnormality;
wherein:
the notifier has a display unit (40) that includes a light source, and a sounder adapted to generate an alert sound;
the display unit is adapted to execute a first mode and a second mode alternately and one or more times respectively, so that the display unit makes the light source emit light with a first intensity in the first mode, and the display unit either makes the light source emit light with a second intensity lower in power consumption than the first intensity, or makes the light source not emit light in the second mode;
the sounder is adapted to execute a third mode and a fourth mode alternately and
one or more times respectively, so that the sounder generates the alert sound with a third intensity in the third mode, and the sounder either generates the alert sound with a fourth intensity lower in power consumption than the third intensity, or does not generate the alert sound in the fourth mode; **characterized in that** at least a half part of each of periods in which the first mode is executed overlaps timewise with a corresponding one of periods in which the fourth mode is executed; and
at least a half part of each of periods in which the third mode is executed overlaps timewise with a corresponding one of periods in which the second mode is executed.

2. An automated external defibrillator according to Claim 1, wherein:
at least a nine tenth part of the period in which the first mode is executed overlaps timewise with the period in which the fourth mode is executed; and
at least a nine tenth part of the period in which the third mode is executed overlaps timewise with the period in which the second mode is executed.

3. An automated external defibrillator according to Claim 1, wherein:
the period in which the first mode is executed and the period in which
the third mode is executed do not overlap timewise with each other.

4. An automated external defibrillator according to any one of Claims 1 to 3, wherein:
each of the period in which the first mode is executed, the period in which the second mode is executed, the period in which the third mode is executed, and the period in which the fourth mode is executed has a length not more than 1,000 milliseconds.

5. An automated external defibrillator according to any one of Claims 1 to 4, wherein:
the second mode is a mode in which the display unit does not make the light source emit light;
the fourth mode is a mode in which the sounder does not generate the alert sound;
the sounder executes the fourth mode in the period in which the display unit executes the first mode; and
the display unit executes the second mode in the period in which the sounder executes the third mode.

6. A method for notification about an abnormality of an automated external defibrillator, the method making the automated external defibrillator execute
a detection step of detecting an abnormality of the automated external defibrillator; and
a notification step of notifying a user of the detection of the abnormality within a predetermined period when the abnormality has been detected in the detection step; wherein:
the notification step has a displaying step of making a display by a light source, and a sounding step of generating an alert sound;
the displaying step is a step of executing a first mode and a second mode alternately and one or more times respectively, so that the light source is made to emit light with a first intensity in the first mode, and
the light source is either made to emit light with a second intensity lower in power consumption than the first intensity, or made not to emit light in the second mode;
the sounding step is a step of executing a third mode and a fourth mode alternately and one or more times respectively, so that the alert sound is generated with a third intensity in the third mode, and the alert sound is either generated with a fourth intensity lower in power consumption than the third intensity, or not generated in the fourth mode;
**characterized in that** at least a half part of each of periods in which the first mode is executed overlaps timewise with a corresponding one of periods in which the fourth mode is executed; and
at least a half part of each of periods in which the third mode is executed overlaps timewise with a corresponding one of periods in which the second mode is executed.

## Patentansprüche

1. Automatisierter externer Defibrillator (I), der umfasst:
einen Detektor (10), der so eingerichtet ist, dass er eine Anomalie des automatischen externen Defibrillator erfasst; sowie
eine Benachrichtigungseinrichtung, die so eingerichtet ist, dass sie einen Benutzer, wenn der Detektor die Anomalie erfasst hat, innerhalb einer vorgegebenen Periode über die Erfassung der Anomalie benachrichtigt;
wobei:
die Benachrichtigungseinrichtung eine Anzeige-Einheit (40) aufweist, die eine Lichtquelle sowie einen akustischen Signalgeber enthält, der so eingerichtet ist, dass er einen Warnton erzeugt;
die Anzeige-Einheit so eingerichtet ist, dass sie abwechselnd einen ersten und einen zweiten Modus jeweils ein- bzw. mehrmals so ausführt, dass die Anzeige-Einheit die Lichtquelle in dem ersten Modus veranlasst, Licht mit einer ersten Intensität zu emittieren, und die Anzeige-Einheit die Lichtquelle in dem zweiten Modus entweder veranlasst, Licht mit einer zweiten Intensität zu emittieren, bei der der Energieverbrauch niedriger ist als bei der ersten Intensität, oder die Lichtquelle in dem zweiten Modus veranlasst, kein Licht zu emittieren;
der akustische Signalgeber so eingerichtet ist, dass er abwechselnd einen dritten Modus und einen vierten Modus jeweils ein- bzw. mehrmals so ausführt, dass der akustische Signalgeber in dem dritten Modus den Warnton mit einer dritten Intensität erzeugt und der akustische Signalgeber in dem vierten Modus entweder den Warnton mit einer vierten Intensität erzeugt, bei der der Energieverbrauch niedriger ist als bei der dritten Intensität, oder den Warnton nicht erzeugt;
**dadurch gekennzeichnet, dass**
wenigstens eine Hälfte aller Perioden, in denen der erste Modus ausgeführt wird, sich zeitlich mit einer entsprechenden der Perioden überlappt, in denen der vierte Modus ausgeführt wird; und
wenigstens eine Hälfte aller Perioden, in denen der dritte Modus ausgeführt wird, sich zeitlich mit einer entsprechenden der Perioden überlappt, in denen der zweite Modus ausgeführt wird.

2. Automatisierter externer Defibrillator nach Anspruch 1, wobei:
wenigstens neun Zehntel der Periode, in der der erste Modus ausgeführt wird, sich zeitlich mit der Periode überlappen, in der der vierte Modus ausgeführt wird; und
wenigstens neun Zehntel der Periode, in der der dritte Modus ausgeführt wird, sich zeitlich mit der Periode überlappen, in der der zweite Modus ausgeführt wird.

3. Automatisierter externer Defibrillator nach Anspruch 1, wobei:
die Periode, in der der erste Modus ausgeführt wird, und die Periode, in der der dritte Modus ausgeführt wird, einander zeitlich nicht überlappen.

4. Automatisierter externer Defibrillator nach einem der Ansprüche 1 bis 3, wobei:
jede von der Periode, in der der erste Modus ausgeführt wird, der Periode, in der der zweite Modus ausgeführt wird, der Periode, in der der dritte Modus ausgeführt wird, sowie der Periode, in der der vierte Modus ausgeführt wird, jeweils eine Länge von nicht mehr als 1.000 Millisekunden hat.

5. Automatisierter externer Defibrillator nach einem der Ansprüche 1 bis 4, wobei:
der zweite Modus ein Modus ist, in dem die Anzeige-Einheit die Lichtquelle nicht veranlasst, Licht zu emittieren
der vierte Modus ein Modus ist, in dem der akustische Signalgeber den Warnton nicht erzeugt;
der akustische Signalgeber den vierten Modus in der Periode ausführt, in der die Anzeige-Einheit den ersten Modus ausführt; und
die Anzeige-Einheit den zweiten Modus in der Periode ausführt, in der der akustische Signalgeber den dritten Modus ausführt.

6. Verfahren für Benachrichtigung über eine Anomalie eines automatisierten externen Defibrillators, wobei mit dem Verfahren der automatisierte externe Defibrillator veranlasst wird, einen Erfassungsschritt zum Erfassen einer Anomalie des automatisierten externen Defibrillators; sowie
einen Benachrichtigungsschritt zum Benachrichtigen eines Benutzers über die Erfassung der Anomalie innerhalb einer vorgegebenen Periode auszuführen, wenn die Anomalie in dem Erfassungsschritt erfasst worden ist; wobei:
der Benachrichtigungsschritt einen Anzeigeschritt, in dem eine Anzeige durch eine Lichtquelle veranlasst wird, sowie einen Tonerzeugungsschritt aufweist, in dem ein Warnton erzeugt wird;
der Anzeigeschritt ein Schritt ist, in dem abwechselnd ein erster Modus und ein zweiter Modus jeweils ein- bzw. mehrmals so ausgeführt werden, dass die Lichtquelle in dem ersten Modus veranlasst wird, Licht mit einer ersten Intensität zu emittieren, und die Lichtquelle in dem zweiten Modus entweder veranlasst wird, Licht mit einer zweiten Intensität zu emittieren, bei der der Energieverbrauch niedriger ist als bei der ersten Intensität, oder veranlasst wird, kein Licht zu emittieren;
der Tonerzeugungsschritt ein Schritt ist, in dem ein dritter Modus und ein vierter Modus abwechselnd und jeweils ein- bzw. mehrmals so ausgeführt werden, dass der Warnton in dem dritten Modus mit einer dritten Intensität erzeugt wird und der Warnton in dem vierten Modus entweder mit einer vierten Intensität erzeugt wird, bei der der Energieverbrauch niedriger ist als bei der dritten Intensität, oder nicht erzeugt wird;
**dadurch gekennzeichnet, dass**
wenigstens eine Hälfte aller Perioden, in denen der erste Modus ausgeführt wird, sich zeitlich mit einer entsprechenden der Perioden überlappt, in denen der vierte Modus ausgeführt wird; und
wenigstens eine Hälfte aller Perioden, in denen der dritte Modus ausgeführt wird, sich zeitlich mit einer entsprechenden der Perioden überlappt, in denen der zweite Modus ausgeführt wird.

## Revendications

1. Défibrillateur externe automatisé (1) comprenant :
un détecteur (10) adapté pour détecter une anomalie du défibrillateur externe automatisé ; et
un dispositif d'alerte adapté pour notifier à un utilisateur la détection de l'anomalie à l'intérieur d'une période prédéterminée lorsque le détecteur a détecté l'anomalie :
le dispositif d'alerte ayant une unité d'affichage (40) qui inclut une source de lumière, et un dispositif d'émission de son adapté pour générer un son de notification ;
l'unité d'affichage étant adaptée pour exécuter un premier mode et un second mode alternativement et une ou plusieurs fois respectivement, de sorte que l'unité d'affichage fait émettre de la lumière par la source de lumière avec une première intensité dans le premier mode, et l'unité d'affichage soit fait émettre de la lumière par la source de lumière avec une seconde intensité inférieure en termes de consommation d'énergie par rapport à la première intensité, soit ne fait pas émettre de lumière par la source de lumière dans le second mode ;
le dispositif d'émission de son étant adapté pour exécuter un troisième mode et un quatrième mode alternativement et une ou plusieurs fois respectivement, de sorte que le dispositif d'émission de son génère le son de notification avec une troisième intensité dans le troisième mode, et le dispositif d'émission de son soit génère le son de notification avec une quatrième intensité inférieure en termes de consommation d'énergie par rapport à la troisième intensité, soit ne génère pas de son de notification dans le quatrième mode ;
**caractérisé en ce qu'**au moins une demi-partie de chacune des périodes dans lesquelles le premier mode est exécuté chevauche dans le temps l'une correspondante des périodes dans lesquelles le quatrième mode est exécuté ; et
au moins une demi-partie de chacune des périodes dans lesquelles le troisième mode est exécuté chevauche dans le temps l'une correspondante des périodes dans lesquelles le second mode est exécuté.

2. Défibrillateur externe automatisé selon la revendication 1 :
au moins neuf dixièmes de la période dans laquelle le premier mode est exécuté chevauchent dans le temps la période dans laquelle le quatrième mode est exécuté ; et
au moins neuf dixièmes de la période dans laquelle le troisième mode est exécuté chevauchent dans le temps la période dans laquelle le second mode est exécuté.

3. Défibrillateur externe automatisé selon la revendication 1 :
la période dans laquelle le premier mode est exécuté et la période dans laquelle le troisième mode est exécuté ne se chevauchent pas l'une l'autre dans le temps.

4. Défibrillateur externe automatisé selon l'une quelconque des revendications 1 à 3 :
chacune de la période dans laquelle le premier mode est exécuté, la période dans laquelle le second mode est exécuté, la période dans laquelle le troisième mode est exécuté, et la période dans laquelle le quatrième mode est exécuté présente une longueur non supérieure à 1 000 millisecondes.

5. Défibrillateur externe automatisé selon l'une quelconque des revendications 1 à 4 :
le second mode étant un mode dans lequel l'unité d'affichage ne fait pas émettre de lumière par la source de lumière ;
le quatrième mode étant un mode dans lequel le dispositif d'émission de son ne génère pas de son de notification ;
le dispositif d'émission de son exécutant le quatrième mode dans la période dans laquelle l'unité d'affichage exécute le premier mode ; et
l'unité d'affichage exécutant le second mode dans la période dans laquelle le dispositif d'émission de son exécute le troisième mode.

6. Procédé d'alerte concernant une anomalie d'un défibrillateur externe automatisé, le procédé faisant exécuter au défibrillateur externe automatisé une étape de détection afin de détecter une anomalie du défibrillateur externe automatisé ; et
une étape d'alerte afin de notifier à un utilisateur la détection de l'anomalie à l'intérieur d'une période prédéterminée lorsque l'anomalie a été détectée dans l'étape de détection :
l'étape d'alerte ayant une étape d'affichage permettant un affichage par une source de lumière, et une étape d'émission de son afin de générer un son de notification ;
l'étape d'affichage étant une étape consistant à exécuter un premier mode et un second mode alternativement et une ou plusieurs fois respectivement, de sorte que la source de lumière est amenée à émettre de la lumière avec une première intensité dans le premier mode, et la source de lumière est soit amenée à émettre de la lumière avec une seconde intensité inférieure en termes de consommation d'énergie par rapport à la première intensité, soit n'est pas amenée à émettre de lumière dans le second mode ;
l'étape d'émission de son est une étape d'exécution d'un troisième mode et d'un quatrième mode alternativement et une ou plusieurs fois respectivement, de sorte que le son de notification est généré avec une troisième intensité dans le troisième mode, et que le son de notification est soit généré avec une quatrième intensité inférieure en termes de consommation d'énergie par rapport à la troisième intensité, soit n'est pas généré dans le quatrième mode ;
**caractérisé en ce qu'**au moins une demi-partie de chacune des périodes dans lesquelles le premier mode est exécuté chevauche dans le temps l'une correspondante des périodes dans lesquelles le quatrième mode est exécuté ; et
au moins une demi-partie de chacune des périodes dans lesquelles le troisième mode est exécuté chevauche dans le temps l'une correspondante des périodes dans lesquelles le second mode est exécuté.
